# EUROPEAN PATENT SPECIFICATION

(11) **EP 1 698 704 B1**
(45) Date of publication and mention of the grant of the patent: **22.09.2010**
(21) Application number: 05101533.7
(22) Date of filing: 01.03.2005
(51) Int. Cl.: C12R 1/225, C12N 1/00, A23L 1/302

(54) **Bacteria that naturally overproduce folate**
Bakterien die natürlicherweise Folat überproduzieren
Bacteries qui surproduisent naturellement le folate.

(43) Date of publication of application: 06.09.2006
(73) Proprietor: Campina Nederland Holding B.V., 5301 LB Zaltbommel (NL)
(72) Inventor: Wegkamp, Hendrikus, 6702 BS, Wageningen (NL); Smid, Eilt Johannes, 6708 LG, Wageningen (NL); De Vos, Willem Meindert, 6721 SJ, Bennekom (NL)
(74) Representative: van Westenbrugge, Andries

(56) References cited:
- TAMURA TSUNENOBU ET AL: "The form of folate affects the mechanisms of methotrexate resistance in Enterococcus faecium" MICROBIOLOGY (READING), vol. 143, no. 8, 1997, pages 2639-2646, XP002332877 ISSN: 1350-0872
- SYBESMA WILBERT ET AL: "Effects of cultivation conditions on folate production by lactic acid bacteria" APPLIED AND ENVIRONMENTAL MICROBIOLOGY, WASHINGTON,DC, US, vol. 69, no. 8, August 2003 (2003-08), pages 4542-4548, XP002308563 ISSN: 0099-2240
- SYBESMA WILBERT ET AL: "Increased production of folate by metabolic engineering of Lactococcus lactis." APPLIED AND ENVIRONMENTAL MICROBIOLOGY, vol. 69, no. 6, June 2003 (2003-06), pages 3069-3076, XP002332878 ISSN: 0099-2240
- WEGKAMP ARNO ET AL: "Transformation of folate-consuming Lactobacillus gasseri into a folate producer." APPLIED AND ENVIRONMENTAL MICROBIOLOGY. MAY 2004, vol. 70, no. 5, May 2004 (2004-05), pages 3146-3148, XP002332879 ISSN: 0099-2240

## Description

### FIELD OF THE INVENTION

The present invention relates to methods for selecting mutant bacteria producing increased folate levels. In these methods the antifolate methotrexate (MTX) is used as selection agent. Provided are also food and food supplement compositions comprising the mutant bacteria or derivatives or extracts of the bacteria.

### BACKGROUND OF THE INVENTION

Folate analogues and in particular inhibitors of dihydrofolate reductase have been the topic of research for many years because of their potential role in cancer chemotherapy and as agents against the malaria parasite. One of the first folate analogues described in literature is methotrexate from Lederle Laboratories. This compound has already been used in 1948 for the treatment of acute leukemia (Hitchings, G. H., Jr., 1989, In Vitro Cell Dev Biol 25:303-10).

The activity of methotrexate (MTX) is based on competitive inhibition of the target enzyme dihydrofolate reductase. This enzyme is needed for the production and recycling of tetrahydrofolate (THF) using dihydrofolate (DHF) as a substrate. The specificity of the methotrexate can be explained by small structural differences of dihydrofolate reductases in different species (Chang et al., 1978, Nature 275:617-24). Methotrexate prevents the cell from producing sufficient tetrahydrofolate for the biosynthesis of methionine, DNA and RNA. However in the presence of purines, thymidine, glycine, methionine and pantothenic acid (all metabolites that need tetrahydrofolate for their own biosynthesis) the demand of cells to synthesize tetrahydrofolate can be decreased (Harvey, R. J., 1973, J Bacteriol 114:309-22). Nevertheless cells will always be in some way hampered in growth by MTX, because THF is essential for formation of methionyl-tRNA^{fmet}, a compound that is indispensable for the initiation of protein synthesis, regardless of the presence of folate dependent metabolites (Baumstark et al., 1977, J Bacteriol 129:457-71).

Lactic acid bacteria such as *Lactococcus lactis, Lactobacillus plantarum* and *Lactobacillus casei* show a high degree of variability in their response to methotrexate and another folate analogue, trimethoprim. *L. lactis* is known be insensitive to both antifolates (Leszczynska et al., 1995, Appl Environ Microbiol 61:561-6). It has been shown for numerous organisms that prolonged exposure to antifolates results in selection of resistant cell types. Several underlying mechanisms have been described to explain the resistance mechanism of both eukaryote cell lines as well as bacteria to methotrexate (MTX). Tamura et al. (1997, Microbiology 143: 2639-46) investigated the mechanisms leading MTX resistance in *Enterococcus hirae.* They found several phenotypic effects including (i) increased folic acid reductase (FAR) activity (ii) increased dihydrofolate reductase (DHFR) activity, (iii) decreased synthesis and intracellular retention of MTX containing two glutamyl residues, (iv) decreased uptake of MTX accompanied by decreased uptake of folates; and (v) reduction of folate-binding capacity. The form of folate present in the media during the development of resistance affected DHFR and FAR activities and the transport of folates.

However, although it is known that exposure to MTX can be used to select cells having increased resistance to MTX, no link between MTX resistance and folate levels of the cells has been disclosed in the prior art. In contrast to the MTX resistance mechanisms described above, the present inventors have surprisingly found that bacterial cells have another way of generating MTX resistance. This mechanism is the production of high levels of folate. This finding can be exploited in the screening and selection of (spontaneous) mutant bacteria producing high folate levels, by using methotrexate as selection agent. Such bacteria, especially food grade bacteria, can be used to fortify food products with folate. Folate is a soluble B vitamin, required for proper cell growth and functioning. Many food products, such as bread, cereals, dairy products, etc. are supplemented with folic acid to ensure adequate intake of folate. The mutant bacteria of the present invention enable fortification of food products with natural folate by for example fermentation using the mutant bacteria.

### DEFINITIONS

"Lactic acid bacteria" as used herein, are bacteria, which produce lactic acid as an end product of fermentation, such as bacteria of the genus *Lactobacillus, Lactococcus, Streptococcus* and *Bifidobacterium.*

A bacterial "strain" or "isolate" is used herein interchangeably and refers to a bacterium which remains genetically unchanged when grown or multiplied. The multiplicity of identical bacteria are included.
A "mutant bacterium" or a "mutant bacterial strain or isolate" refers to a natural (spontaneous, naturally occurring) mutant bacterium or an induced mutant bacterium comprising one or more mutations in its genome (DNA) which are absent in the wild type DNA. An "induced mutant" is a bacterium where the mutation was induced by human treatment, such as treatment with chemical mutagens, UV- or gamma radiation, etc. In contrast, a "natural" or "spontaneous mutant" has not been mutagenized by man. Mutant bacteria are herein non-GMO, i.e. not modified by recombinant DNA technology.
"Wild type strain" or "wild type isolate" refers to the non-mutated form of a bacterium, as found in nature.
"Folate dependent metabolites" refers to metabolites or precursors of folate biosynthesis or catabolism which are able to mask MTX sensitivity of a cell when present in the surrounding medium.
"Inhibitory amount of MTX" refers to the amount required to substantially inhibit growth of a cell. Generally, for prokaryotic cells an amount of at least about 1.25mg/L growth medium is inhibitory for MTX sensitive cells.
"Total folate" refers to the extracellular (secreted) and intracellular folate levels produced by a strain.
"Food-grade" micro-organisms are in particular organisms, which are considered as not harmful, when ingested by a human or animal subject.
The term "comprising" is to be interpreted as specifying the presence of the stated parts, steps or components, but does not exclude the presence of one or more additional parts, steps or components. A composition comprising bacterial strain X, may thus comprise additional strains, other components, etc.
In addition, reference to an element by the indefinite article "a" or "an" does not exclude the possibility that more than one of the element is present, unless the context clearly requires that there be one and only one of the elements. The indefinite article "a" or "an" thus usually means "at least one".

### DETAILED DESCRIPTION OF THE INVENTION

While testing whether recombinant folate overproducing bacteria have modified susceptibility to MTX, it was found that a number of wild type strains of *L. plantarum,* which were incubated for more than about 40 hours on inhibitory amounts of MTX (1,25 mg/L) were not inhibited by MTX, but had a growth rate similar to that on medium lacking MTX. When analysing the folate levels of these strains, approximately 5% of these colonies displayed a significant higher folate production compared to the (non mutated) wild type strain. The total folate pools were 4% to 63% higher compared to (average) wild-type levels. This observation demonstrated that MTX can be used to efficiently select natural (non-GMO) folate overproducing bacteria. The resistance to MTX was maintained, even when the strains were grown for more than 50 generations on medium lacking MTX, which indicated that the resistance is caused by (spontaneous) stable mutations in the bacterial genome.

The same effect, that increased folate production leads to high resistance against MTX, was seen in recombinant *L. plantarum* strains, which were transformed with the complete folate biosynthesis gene cluster under the control of a strong promoter. These recombinant strains produced high levels of folate and were resistant to MTX when grown in a medium lacking folate dependent metabolites. Without limiting the scope of the invention, it is speculated that the intracellular dihydrofolate levels compete with the accumulated MTX for the active site on the enzyme dihydrofolate reductase (DHFR).

This fmding was applied to develop a rapid screening method, based on growth in the presence of MTX, to select folate overproducing bacterial strains. The folate overproducers that were identified in this study displayed a high increase in folate levels compared to the wild type folate producers. The total folate levels ranged from 20 to 60 times the level of the wild type. The intracellular folate pools found in the folate overproducers were 10 to 20 times higher compared to the levels found in the wild type strain. These increased intracellular folate levels cause a decreased sensitivity (i.e. resistance) towards MTX. This explained why growth of the folate overproducing strains was not significantly affected by MTX. Sensitivity of the wild-type towards MTX was only observed when all the metabolites which are associated with folate were omitted from the medium.

### Bacteria according to the invention

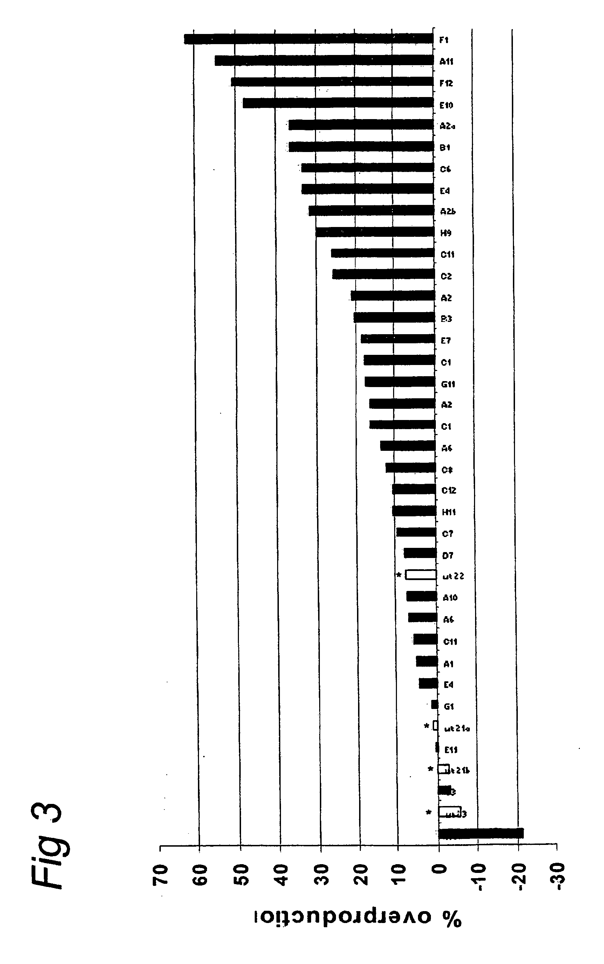

Therefore, in one embodiment of the invention mutant bacteria are provided which comprises an increased amount of intracellular and/or extracellular folate compared to the wild type and which have a growth rate (µ) of at least 0.1 h⁻¹ when grown on medium comprising (at least) 1.25 mg/l methotrexate and lacking folate dependent metabolites (i.e. they are said to be "resistant" to MTX). Preferably under these growth conditions, the growth rate of the mutant bacterium is not significantly different than that of the wild type strain when the wild type strain is grown on the same medium lacking MTX (i.e. "normal growth"). Most importantly, the mutant bacterium shows significant growth at an MTX concentration which is inhibitory to the wild type strain. An "Inhibitory concentration" is the concentration of MTX which results in substantially no growth of the wild type strain. Figure 3, for example, shows that at concentrations of 1.25, 1.5, 2.0 and 2.5 mg/l MTX, the wild type has a growth rate close to zero (µ < 0.05 h⁻¹), while the folate overproducing strains have a growth rate (µ) of above 0.1 h⁻¹, especially around about 0.15 h⁻¹. The growth medium should not be supplemented with folate dependent metabolites, as in their presence no significant difference in MTX resistance can be seen, probably because the wild type strain compensates for its lower folate levels by using the folate metabolites of the medium. Suitable growth medium is for example modified CDM (Chemically Defined Medium), which lacks glycine, inosine, orotic acid, thymidine, guanine, adenine, uracil and xanthine.

Growth rate can be measured by various means. For example, at certain time points following inoculation of the medium with the bacterial strain or isolate spectrophotometric readings can be taken and the growth rate over time calculated.

The mutant bacterium preferably comprises an increased amount of intracellular and/or extracellular folate compared to the wild type strain. Preferably, the amount of total folate is at least about 4%, 5%, 10%, 15%, 20%, 25%, 30%, 40%, 50%, more preferably at least about 60%, 63% (or more) higher than the average amount of intracellular folate of the wild type strain(s). As in some bacterial species the wild type strains show significant variation in intracellular folate levels, it is preferred that a number of different wild type strains (e.g. 2, 3, 4 or more strains) are analysed for intracellular folate levels and the average folate level is calculated.
The total, intracellular or extracellular amount of folate produced by a bacterial isolate or strain can be determined and quantified by methods known in the art, such as the *L*. *casei* microbiological assay described by Sybesma (see example 1.3) or variants thereof, by HPLC analysis or other known methods. A microbiological test may for example use an indicator strain (e.g. an *L. casei* strain), which is grown on folate produced by the test strain. If large amounts of folate are produced by the test strain, the indicator strain grows well, resulting in a high OD measurement after a specified period of growth. In contrast, if low amounts of folate a produced by the test strain, lower OD values will be measured.
The mutant bacterium is preferably a spontaneous (natural) mutant, selected for example using the method according to the invention (see further below). The bacterium is of any species of which the wild type strain shows natural sensitivity to MTX, such as *L. plantarum, L. casei,* or other species of the group of lactic acid bacteria. Being sensitive to MTX means that growth of the bacterium is substantially inhibited by at least 1.25mg/L MTX, when the strain is grown in a medium lacking folate dependent metabolites. Whether a bacterial species shows sensitivity to MTX can be easily tested by growing the bacterium on medium supplemented with MTX and lacking folate dependent metabolites. The bacterium is preferably a food grade bacterium and, in one embodiment, it belongs to a genus selected from the group consisting of *Lactobacillus, Lactococcus, Streptococcus, Bifidobacterium, Leuconostoc and Streptococcus.*

Preferably it is a food grade lactic acid bacterium. It may be a species selected from the group consisting of *Lactobacillus reuteri, L. fermentum, L. acidophilus, L. crispatus, L. gasseri, L. johnsonii, L. casei, L. plantarum, L. paracasei, L. murinus, L. jensenii, L. salivarius, L. minutis, L. brevis, L. gallinarum, L. amylovorus, Lactococcus lactis, Streptococcus thermophilus, Leuconostoc mesenteroides, Lc. lactis, Pediococcus damnosus, P. acidilactici, P. parvulus, Bifidobacterium bifidum, B. longum, B. infantis, B. breve, B. adolescente, B. animalis, B. gallinarum, B. magnum,* and *B. thermophilum.*

In an alternative embodiment the mutant bacterium is an induced mutant having increased folate and being resistant to MTX. Such a mutant may be obtained using the same method as used in the selection of natural mutants (described herein below), except that prior to the screening method a mutagenesis step is included. A mutagenesis step may include one or more known mutagenesis methods, such as exposure to a chemical and/or physical mutagen (e.g. N-methyl-N'-nitro-N-nitrosoguanidine; UV radiation, gamma-radiation, etc.). Following mutagenesis, the bacteria are grown in/on medium comprising inhibitory amounts of MTX and isolates which do grow are selected and analysed for folate content.

In yet another embodiment, the mutant bacteria according to the invention can be characterized by their gene expression pattern. This is illustrated by the fmding that methotrexate resistant *L. plantarum* strains producing at least 50% more folate compared to the wild-type show a 2-fold (or more) overexpression of one or more of the *folC* genes in the genome. The gene *folC* codes for the enzyme dihydrofolatesynthase. In one embodiment the bacteria according to the invention show overexpression compared to the wild-type of one or more genes coding for enzymes of the folate biosynthesis pathway. In addition to the gene directly involved in folate biosynthesis, also higher transcription levels were found for the following genes: FK (coding for fructokinase), POX (pyruvate oxidase), LOX (lactate oxidase) and PDH (pyruvate dehydrogenase). Compared to the wild-type, a significantly lower transcript level was found for: DAK (dihydroxyacetone phosphotransferase or glycerone kinase). Thus, in one embodiment of the invention, the bacterium/bacteria according to the invention comprise significantly enhanced mRNA transcript levels of one or more genes of the group encoding dihydrofolate synthase (EC 6.3.2.17), fructokinase (EC 2.7.1.4), pyruvate oxidase (EC 1.2.3.3), and 6-phospho-β-glucosidase (EC 3.2.1.86). Additionally or alternatively, the bacteria comprise significantly reduced mRNA transcript levels of the gene encoding for dihydroxyacetone phosphotransferase (= glycerine kinase) (EC. 2.7.1.29).

The term "upregulated"or "downregulated" refers to an increased mRNA transcription level or a decreased mRNA transcription level compared to the gene in the wild type strain.
A "significantly enhanced transcript level" or a "significantly enhanced transcription level" refer to an amount of mRNA transcript of at least about 2 fold, preferably at least about 3 fold, or even about 4 fold or more compared to the transcript level found in the wild type. Likewise, a "significantly reduced transcript level" or "transcription level" refers to an amount of mRNA transcript of at least about 2 fold, 3 fold, etc. less than found in the wild type.
mRNA transcript levels of one or more genes can be measured and quantified using routine molecular biology methods, such as for example Northern analysis and quantitative reverse transcriptase (RT-) PCR. As DNA/cDNA sequence information is available for these genes, primers and probes suitable for hybridizing to the target mRNA or cDNA are available and only routine experimentation is required for the analysis of transcript levels. For example, the nucleic acid sequences of *L. plantarum* (the genome of which has been sequenced) may be used to identify (e.g. by *in silico* analysis), clone (e.g. using PCR based methods, hybridization based methods, etc) and/or sequence the orthologous genes from other species of bacteria. The nucleic acid sequences of these genes, or parts thereof, may then be used to make primers or probes for detection and quantification of the target genes.

Optionally, the transcription pattern (i.e. the enhanced or reduced transcript levels of one or more of the genes mentioned above) may be used as a selection criterion. For example, bacteria may first be screened for altered expression levels in a first screen, and subsequently analysed for methotrexate resistance and/or folate levels (as described below). Likewise, it may be used as an additional screening/selection step in the method described below (for example between steps (b) and (c) or after step (d), or it may even replace steps (a) and (b).

The transcription profile of one of the methotrexate resistant strains described herein (*Lactobacillus plantarum* NIZO B2550) has been compared with that of the parental strain (*Lactobacillus plantarum* WCFS1 (the full sequence of *L. plantarum* WCFS1 has been deposited in the EMBL database under accession no. AL935263)). This was done by using whole genome DNA micro-arrays of *Lactobacillus plantarum* WCFS 1. Total mRNA samples of the parental strain and the methotrexate resistant mutant were isolated and subsequently treated with reverse transcriptase to synthesize fluorescently labelled cDNA. The samples of parental strain and mutant were differentially labelled. Subsequently, a mixture of the labelled cDNA samples was hybridized on the DNA-microarray to determine the relative abundance of the mRNA of all genes in the mutant relative to the parental strain of *L. plantarum.*

In one embodiment the mutant bacteria are the bacterial strains (or any derivatives thereof) deposited by NIZO Food Research, P.O. Box 20, 6710 BA Ede, the Netherlands at the CBS (Centraalbureau voor Schimmelcultures, Uppsalalaan 8, 3584 CT Utrecht, The Netherlands) under the Budapest Treaty under Accession number CBS 117120.

### Compositions according to the invention

A further aspect of the invention relates compositions comprising mutant bacteria as described herein above or derivatives or extracts thereof, as well as to methods for the production of such compositions. The bacteria of the invention are cultured under appropriate conditions, optionally recovered from the culture medium and optionally formulated into a composition suitable for the intended use. Methods for the preparation of such compositions are known *per se.*

Preferably, the mutant bacteria, derivatives or extracts thereof, are used to make food or food supplement compositions comprising natural folate.

A preferred composition according to the invention is suitable for consumption by a subject, preferably a human or an animal. Such compositions may be in the form of a food supplement or a whole food or food composition, which besides the bacteria of the invention also contains a suitable food base. A food or food composition is herein understood to include not only solid and semi-solid compositions, but also liquids for human or animal consumption, i.e. a drink or beverage. The food or food composition may be a solid, semi-solid and/or liquid food or food composition, and in particular may be a dairy product, such as a fermented dairy product, including but not limited to a yoghurt, a yoghurt-based drink or buttermilk. Such foods or food compositions may be prepared in a manner known *per se,* e.g. by adding one or more mutant bacteria of the invention to a suitable food or food base, in a suitable amount.

Also food supplements can be made, comprising suitable amounts of one or more mutant bacterial strains, or extracts therefrom (e.g. partially or substantially purified natural folate). Food supplements include for example vitamin tablets, pills, capsules or powders, comprising recommended daily amounts of various vitamins, including folate. The synthetic folic acid normally used in such preparations may be replaced with the natural folate according to the invention.

In a further preferred embodiment, the live bacteria are used in or for the preparation of a food or food composition, e.g. by fermentation. In doing so, the bacteria of the invention may be used in a manner known *per se* for the preparation of such fermented foods or food compositions, e.g. in a manner known *per se* for the preparation of fermented dairy products using lactic acid bacteria. In such methods, the bacterial cells of the invention may be used in addition to the micro-organism usually used, and/or may replace one or more or part of the micro-organism usually used. For example, in the preparation of fermented dairy products such as yoghurt or yoghurt-based drinks, a food grade mutant bacterium of the invention may be added to or used as part of a starter culture or may be suitably added during such a fermentation. The "growth medium" of the bacteria in this case is a food-grade medium, such as milk-based.

In a further embodiment the bacteria used may be dead (e.g. lysed) or non-viable, or lyophilized.

The compositions manufactured using one or more mutant bacterial strains according to the invention or supplemented with a suitable amount of one or more of the strains, comprise an enhanced amount of natural folate, produced by the bacteria. One or more strains which are high overproducers of folate may be mixed or may be added sequentially to the composition or during its production. The total amount of folate present in the final product can be analysed using for example HPLC analysis.

### Methods and uses according to the invention

Methotrexate is used for the selection of bacteria having increased intracellular and increased total folate levels.

In one embodiment a method for selecting bacteria having increased intracellular and/or total folate levels is provided. The method comprising the steps of:
(a) growing bacteria on (or in) medium comprising methotrexate, wherein the medium is not supplemented with folate dependent metabolites,
(b) selecting bacteria having a growth rate (µ) of at least 0.1 per hour,
(c) determining the folate level of the bacteria selected in step b
(d) and selecting bacteria from having increased folate levels compared to the wild type or another suitable control,
(e) optionally repeating steps (b), (c) and/or (d).

"Growing" in step (a) may also mean contacting the bacteria with such a medium.
The medium may comprise various concentrations of MTX. For example a first round of selection may be carried out on a relatively low concentration, such as 1.25, 1.5, 2.0, 2.5mg/l medium. Isolates growing on one or more of these concentrations may then be screened directly for folate content or may be subjected to one or more further rounds of MTX selection, using for example a higher concentration of MTX, such as 4, 5, 10 or more mg/liter. The stringency of the selection process can be varied by selecting more stringently, e.g. only isolates with a high growth rate at high concentration, or less stringently, e.g. isolates showing moderate growth at relatively low MTX concentrations. Preferably, in each MTX selection step, the isolates are grown on or in the medium for at least 40 hours, more preferably at least 50 hours or more. Suitable controls, e.g. wild type isolates, should always be included, as the mutants are selected based on the difference in growth between the mutant and the control (e.g. the wild type). The control need not necessarily be a wild type, but can also be a previously selected strain. Optimum incubation temperature, pH of the medium, and other parameters can be easily determined by the skilled person. The exact conditions depend on the bacterial species used.

Preferably, the method is set up in such a way that large numbers of isolates can be screened simultaneously, e.g. in 96-well microtitre plates or the like.

The method can be applied on all bacteria which show a natural sensitivity for methotrexate. Using the folate analogue methotrexate, one can select and isolate efficiently natural folate overproducing bacteria which can be used for the production of fermented food products with increased folate concentration (natural fortification or fermentation fortification) as described above.

Any bacterium obtainable according to the method and having significantly increased folate levels is comprised herein.

Unless stated otherwise, the practice of the invention will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

### Figure legends

Figure 1:
   Folate production of the *L. plantarum* wild type strain (1) and the *L. plantarum* strains harbouring pNZ7019 (2) and pNZ7021 (3) cultivated on CDM. Legend: extra-cellular folate levels; (white bar), intracellular folate levels; (grey bar), total folate levels; (black bar).
Figure 2:
   The growth rate of the wild type *L. plantarum* strain (1), and of two *L. plantarum* strains harbouring the vector pNZ7019 (2) and pNZ7021 (3). The growth performance of the three *L. plantarum* strains was tested on CDM (a) and the modified CDM (b), containing an increasing concentration of methotrexate 0, 0.3125, 0.625, 1.25 and 2.5 mg/L (MTX).
Figure 3:
   Folate production levels of 15 folate overproducing strains, selected on MTX resistance. The folate production of the 15 strains is compared to the wild type folate production

### EXAMPLES

### 1. Materials and methods

### 1.1 Strains and cultivation conditions

Wild type *L. plantarum* WCFS1 strains and its derivatives were grown at 37 °C on Chemically Defined Media (CDM), containing 19 g/L β-phosphoglycerate (6) or modified CDM. In this modified CDM, glycine, inosine, orotic acid, thymidine, guanine, adenine, uracil and xantine were omitted from the normal CDM. Additionally 8 ng/ml chloroamphenicol was added to all the media used. Methotrexate (Sigma, aminopterin) was added to both types of CDM, in concentrations ranging from 0 to 10 mg/l.

### 1.2 DNA techniques, constructing of plasmids and transformations

The plasmids used in this study are listed in Table 1.

**Table 1.**

| Plasmid | Relevant characteristics |
|---|---|
| pNZ8148 | Cm^{r}; inducible expression vector carrying the *nisA* promoter |
| pNZ7019 | Cm^{r}; pNZ7017 derivative containing the folate gene cluster of *L. lactis* behind the *pepN* promoter (Wegkamp et al, Appl Environ Microbiol. 2004, 70:3146-3148) |
| pNZ7020 | Cmr; pNZ8148 derivative containing the pepN promoter instead of the *nisA* promoter |
| pNZ7020a | Cmr; PNZ7020 derivative containing a part of the folate gene cluster of *L. plantarum, folB* gene to a truncated *folP* gene |
| pNZ7021 | Cm^{r}; pNZ7020a derivative containing the complete folate gene cluster of *L. plantarum folB* to *folP* |

The vector pNZ8148 is an empty vector containing the nisin inducible promoter, a multiple cloning site, and a chloramphenicol resistance marker. The pNZ8148 derivatives and the *L. plantarum* genomic DNA was isolated using established procedures. PCR was performed using Pfx DNA polymerase (Invitrogen, Paisley, UK) in a Mastercycler PCR apparatus (Eppendorf, Hamburg, Germany) using the following cycles: denaturation at 94 °C for 30 s (3 minutes the first cycle), annealing at 45 °C for 25 s, and elongation at 68 °C for 1 minute per 1000 bps for a total of 30 cycles.
The vector pNZ8148 was digested using SphI and Bg1II, thereby excising the nisin promoter. In a parallel experiment the constitutive promoter pepN of *L. lactis* (Van Alen-Boerrigter et al., 1991. Appl. Environ. Microbiol. 57:2555-61) was digested from the vector pNZ7017 using SphI and Bg1II. Hereafter both fragments were ligated using T4 DNA ligase (Invitrogen), the resulting plasmid was designated as vector pNZ7020. The folate gene cluster *of L. plantarum* was amplified from chromosomal DNA using the following primers: (i) folBKpn-F (5'-GAAAGAGGCTGGGTACCATTATGGGCATGATTC-3'), which was extended at the 5' with a KpnI restrictions site, thereby overlapping the start codon of *the folB* gene and (ii) the reverse primer LpfPxba-R (5'-CTTAACCCCATCTAGACGTAATATCG-3') which was extended at the 5' end to create an XbaI restriction site, allowing a fusion between the stop codon of *folC* and the vector. The amplified DNA fragment was digested with XbaI and KpnI, resulting in a truncated version of the *folB-folP* gene cluster, (*folP* contains an XbaI restriction site). The vector pNZ7020 was also digested with XbaI and KpnI, hereafter were both fragments ligated with T4 DNA ligase, the resulting vector was designated as pNZ7020a. The missing part of the *folP* gene was amplified by PCR using the following primers lpfP-F (5'-CATGGCATCGATATTGAACGAATTG-3') and the lpfPxba-R primer (5'-CTTAACCCCATCTAGACGTAATATCG-3'). The amplified DNA was digested with XbaI. Then the vector pNZ7020a was digested with XbaI and subsequently phosphorilised using Alkaline phosphatase (Pharmacia Biotech) to prevent self-ligation. Both XbaI digested pieces of DNA were ligated using T4 DNA ligase, the orientation of the inserted fragment was checked using PCR. The resulting vector was named pNZ7021. The three vectors listed in table 1 where, hereafter transferred into competent cells of *L. lactis* NZ9000 in an *ylgG* background. Subsequently, midiprep of the three vectors were transformed into *L. plantarum* using established procedures.

### 1.3 Folate quantification using the microbiological assay

The folate levels of the three genetically engineered *L. plantarum* strains were analyzed using the *Lactobacillus casei* microbiological assay as described previously (Sybesma et al., 2003. Appl. Environ. Microbiol. 69:3069-76.)

### 1.4 Monitoring growth of the strains

Microtiter plates were used to determine the growth rate of the three *L. plantarum* strains harbouring the different vectors. The growth of the three strains was monitored in a 96 well microtiter plate for 35 hours using a spectrophotometer (SPECTRAmax®, Molecular Devices, Sunnyvale, CA, USA). The growth was tested on the two types of media, and each construct was analyzed in triplicate on both media for the following MTX concentrations: 0, 0.3125, 0.625, 1.25 and 2.5 mg/L.

### 1.5 Selection for natural folate overproducers

The wild type *L. plantarum* WCFS1 containing pNZ8148 was again cultivated in modified CDM containing 2.5 mg/L MTX, but this growth medium was split-up into 96 wells of the microtiter plates. This microtiter plate was incubated for 3 days, and hereafter aliquots from each of the 96 wells were dispersed into two fresh 96 well microtiter plate containing the modified CDM with this time 10 mg/L MTX. Through each step the original microtiter plate was mixed with 60 % glycerol and stored in a -80 °C freezer. The glycerol stored microtiter plates were used to inoculate the mutated or adapted cells on fresh modified CDM medium. The folate levels of these 288 MTX resistance cultures were checked using a quick screening method. This method is based on the normal microbiological assay as described previously, however some changes were adopted. In this quick screening method the folate produced by the wild type is compared, to the folate produced by the mutant strains. Cultivation of the indicator strain on the folate produced by the wild type leads to a relative low optical density. The optical density of the indicator strain on folate overproducers will be higher then of the wild type. Strains producing an increased optical density of the indicator strain were reanalyzed in by using the extended *Lb. casei* microbiological assay.

### 2. Results

### 2.1 Recombinant strains

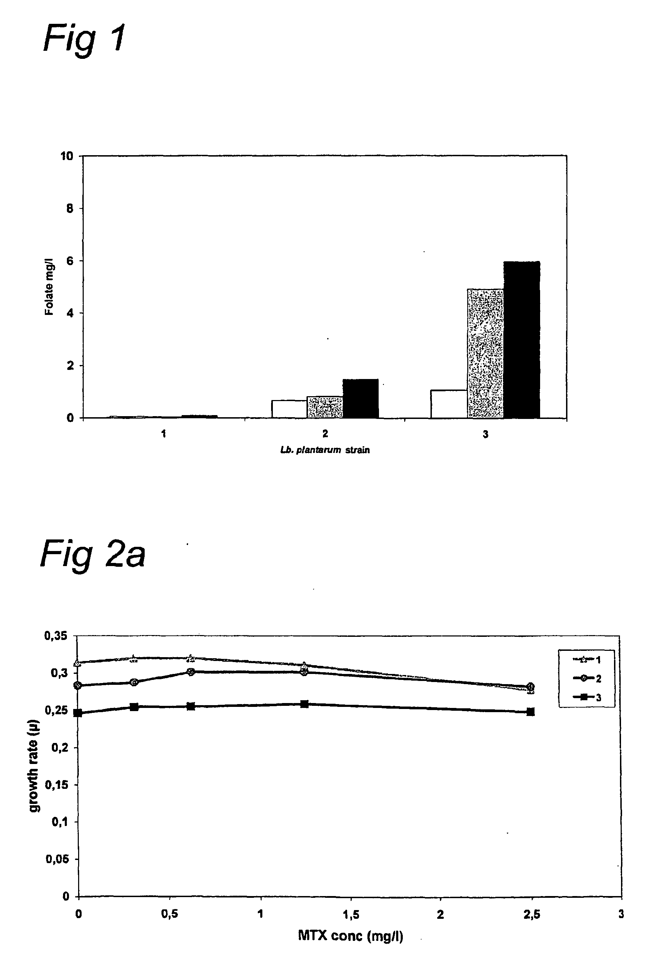

The folate production levels *of L. plantarum* wild type carrying pNZ8148 and the two genetic constructs pNZ7019 and pNZ7021 were studied on normal CDM medium. Folate production *of L. plantarum* WCFS1 strain containing the empty vector pNZ8148 was found to be 100 ng folate/ml culture (Fig 1). *L. plantarum* carrying pNZ7019 (the folate biosynthesis gene cluster of *L. lactis)* produces up to 2000 ng/ml folate in the culture (Fig. 1). This corresponds to approximately 20 times overproduction compared to the wild type. Transformation of *L plantarum* WCFS1 with vector pNZ7021, carrying the folate biosynthesis gene cluster of *L. plantarum,* results in a strain producing folate levels of 6000 ng/ml culture (Fig. 1).

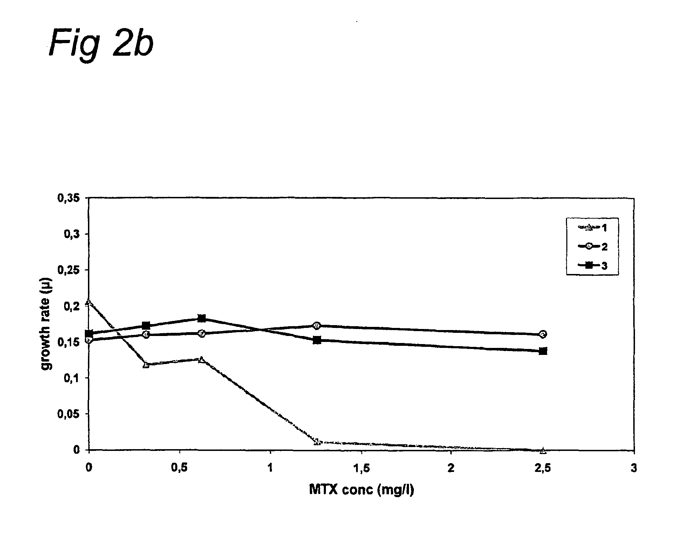

Next, the specific growth rates of the two folate overproducing strains and the wild type strain, were compared on CDM with and without folate dependent metabolites in the presence of a range of methotrexate (MTX) concentrations (Fig. 2). In the presence of the folate dependent metabolites, none of the tested strains was significantly inhibited by MTX (Fig. 2a). The growth rate *of L. plantarum* carrying plasmid pNZ7021 is lower compared to the other two strains, at all tested MTX concentrations. On a medium in the folate dependent metabolites are absent, a completely different picture arises. On this medium, the folate overproducers are clearly more resistant to MTX compared to the wild type strain (Fig 2b). At a concentration of 2.5 mg/L MTX, complete growth inhibition of the wild type strain was observed in the modified CDM, while both folate overproducing strains grew well on this medium. Between 0 and 2.5 mg/L MTX, dose dependent growth inhibition was observed for the wild type *L. plantarum* strain. These results show that, if analysed in a proper growth medium, folate overproduction leads to MTX resistance. Therefore, MTX can be used to discriminate folate overproducing strains from wild type strains.

### 2.2 Wild type strains

Prolonged incubation (>40 hours) of the wild type *L. plantarum* WCFS1 on modified CDM supplemented with 2.5 mg/L MTX, resulted in significant growth of the culture. If this culture was diluted in fresh modified CDM supplemented with 2.5 mg/L MTX, immediate growth of the culture was observed. Single colonies were isolated from this culture to test if natural mutants with increased MTX resistance were selected or if some kind of temporary adaptation takes place. Modified CDM containing 2.5 mg/l MTX was inoculated with a single colony derived from an MTX resistant culture. Next, the growth medium was split into two cultures; one culture was grown for 50 generation in the modified CDM with 2.5 mg/l MTX, the other culture was grown for 50 generations in the same medium in the absence of MTX. After the 50 generation both cultures where transferred again to modified CDM containing the 2.5 mg/l MTX. Both strains with a different history revealed a comparable growth rate on this medium indicating that a stable mutation causes the MTX resistance phenotype.
We isolated 288 colonies from cultures containing 2.5 mg/L or 10 mg/L MTX.

These colonies were analysed for folate overproduction using a rapid pre-screening method. Based of this rapid screen, 5 out of 288 colonies gave raise to cultures with higher folate levels compared to the wild type. These 5 cultures were subsequently plated on modified CDM supplemented with 2.5 mg/L MTX. From each plate 3 single colonies were isolated, stored and analyzed again for folate production in the following way. Folate levels in cultures derived from 15 positive colonies were monitored (Fig. 3). The average folate levels are 20% to 60% higher than that observed in the wild type. To confirm that all colonies were still *L. plantarum,* we have performed RAPD analyses. All colonies tested showed a high similarity with the parental strain.

### 2.3. Transcription profiling.

Using oligonucleotide microarray technology for genome-wide transcription profiling, we have identified genes in the methotrexate resistant mutant *L. plantarum* NIZO B2550 (derived from *L. plantarum* WCFS1) which are specifically up- or down regulated compared to the wild-type. Cultures *of L. plantarum* WCFS1 and NIZO B2550 were grown on modified CDM and at a turbidity (OD₆₀₀) of 1, cells were harvested and mRNA was isolated. The mRNA samples were processed for analysis on DNA micro-arrays. The relative abundance of specific mRNA's was expressed as the fold change in expression of genes in the methotrexate resistant strain compared to the corresponding genes in the parental strain. Analysis of the transcriptome profile reveals that in comparison with the parental strain, *folC1,* the gene coding for a dihydrofolate synthase is specifically upregulated 4-fold (see Table 2) in the methotrexate resistant mutant compared to the wild-type. This enzyme couples L-glutamate to dihydropteroate to form dihydrofolate, which is a structural analogue of methotrexate and serves as the substrate for the enzyme dihydrofolate reductase. More dihydrofolate synthase through overexpression of *folC1* is expected to lead to higher intracellular dihydrofolate pools, which in turn could compete with methotrexate for conversion by dihydrofolate reductase. This observation reveals the mechanism of methotrexate resistance in the isolated mutants. It also explains why these mutants produce higher levels of folate in the absence of methotrexate.
*L. plantarum* WCFS1 has two *folC* genes *(folC1* and *folC2).* Interestingly, only *folC1* is upregulated in the methotrexate resistant mutant. This gene is not located in a functional cluster, this in contrast to *folC2,* which is located in the folate biosynthesis cluster.
In addition to *folC1,* a number of other metabolic genes (coding for fructokinase, pyruvate oxidase and 6-phospho-beta-glucosidase) were found to be upregulated in the methotrexate resistant strain (see Table 2). Three genes *(dak1A, dak1B* and *dak2)* coding for a glycerone kinase (dihydroxyacetone phosphotransferase) were found to down-regulated in the mutant as compared to the parental strain.

**Table 2. Genes specifically up- and down regulated in L. plantarum NIZO B2550 compared to L. plantarum WCFS1.**

| Gene | Locus on chromosome | Enzyme | Fold change |
|---|---|---|---|
| Up regulated | | | |
| *folC1* | Lp_2321 | Dihydrofolate synthase | 4.0 |
| *folC2* | Lp_3296 | Dihydrofolate synthase | 1.1 |
| *sacK1* | Lp_0184 | Fructokinase | 10.2 |
| *sacK2* | Lp_3637 | Fructokinase | 1.2 |
| *pox1* | Lp_3587 | Pyruvate oxidase | 2.1 |
| *pox3* | Lp_2629 | Pyruvate oxidase | 1.6 |
| *pox4* | Lp_0849 | Pyruvate oxidase | 4.2 |
| *pox5* | Lp_3589 | Pyruvate oxidase | 2.7 |
| *pbg6* | Lp_3011 | 6-phospho-β-glucosidase | 6.1 |

| Down regulated | | | |
|---|---|---|---|
| *dak1A* | Lp_0166 | Glycerone kinase | 0.11 |
| *dak1B* | Lp_0168 | Glycerone kinase | 0.13 |
| *dak2* | Lp_0169 | Glycerone kinase | 0.29 |

## Claims

1. A mutant bacterium, **characterized in that** the bacterium comprises an increased amount of intracellular and/or extracellular folate compared to the wild type and having a growth rate of at least 0.1µ per hour when grown on medium comprising 1.25 mg/l methotrexate and lacking folate dependent metabolites, said folate dependent metabolites being metabolites or precursors of folate biosynthesis or catabolism which are able to mask methotrexate sensitivity of a cell when present in the surrounding medium.

2. The bacterium according to claim 1, wherein said bacterium is a naturally occurring mutant or an induced mutant.

3. The bacterium according to claim 1 or 2, wherein said bacterium shows overexpression compared to the wild-type of one or more genes coding for enzymes of the folate biosynthesis pathway.

4. The bacterium according to any of the preceding claims, wherein the expression of one or more of the genes selected from the genes coding for dihydrofolate synthase, fructokinase, pyruvate oxidase and 6-phospho-β-glucosidase are upregulated at least about 2-fold compared to the wildtype.

5. The bacterium according to claim 1, 2 or 4, wherein the expression of the gene coding for dihydroxyacetone phosphotransferase is downregulated at least about 2-fold compared to the wild type.

6. The bacterium according to any of the preceding claims, wherein the amount of total folate of the mutant bacterium is at least 10% higher than that of the wild type strain.

7. The bacterium according to any of the preceding claims, wherein the bacterium is of the species *Lactobacillus plantarum, L. casei, L. reuteri, L. fermentum, L. acidophilus, L. crispatus, L. gasseri, L. johnsonii, L. paracasei, L. murinus, L. jensenii, L. salivarius, L. minutis, L. brevis, L. gallinarum, L. amylovorus, Lactococcus lactis, Streptococcus thermophilus, Leuconostoc mesenteroides, Lc. Lactis, Pediococcus damnosus, P. acidilactici, P. parvulus, Bifidobacterium bifidum, B. longum, B. infantis, B. breve, B. adolescente, B. animalis, B. gallinarum, B. magnum,* and *B. thermophilum.*

8. *Lactobacillus plantarum-strain* CBS 117120.

9. A composition comprising a bacterium according to any of the preceding claims.

10. The composition according to claim 9, wherein the composition is a food composition or food supplement composition.

11. The composition according to claim 10 wherein the food composition is a fermented food composition.

12. Use of methotrexate for the selection of bacteria having increased intracellular and increased total folate levels.

13. A method for selecting bacteria having increased intracellular and/or total folate levels, said method comprising the steps of:
(a) growing bacteria on or in a medium comprising methotrexate, wherein the medium is not supplemented with folate dependent metabolites, said folate dependent metabolites being metabolites or precursors of folate biosynthesis or catabolism which are able to mask methotrexate sensitivity of a cell when present in the surrounding medium,
(b) selecting bacteria having a growth rate (µ) of at least 0.1 per hour,
(c) determining the folate level of the bacteria selected in step (b) and electing bacteria from having increased folate levels compared to the wild type,
(d) optionally repeating steps (a), (b) and/or (c).

14. The method according to claim 13, wherein said medium comprises at least 1.25 mg/l methotrexate and wherein the bacteria are grown on said medium for at least 40 hours.

## Patentansprüche

1. Mutiertes Bakterium, **dadurch gekennzeichnet, dass** das Bakterium im Vergleich zum Wildtyp eine erhöhte Menge an intrazellulärem und/oder extrazellulärem Folat umfasst und eine Wachstumsrate von mindestens 0,1µ pro Stunde hat, wenn es auf einem Medium wächst, das 1,25 mg/l Methotrexat umfasst und dem Folat-abhängige Metabolite fehlen, wobei die Folat-abhängigen Metabolite Metabolite oder Vorläufer der Folatbiosysnthese oder des Folatkatabolismus sind, die geeignet sind, Methotrexatsensitivität einer Zelle zu maskieren, wenn sie im umgebenden Medium vorliegen.

2. Bakterium nach Anspruch 1, wobei das Bakterium eine natürlich vorkommende Mutante oder eine induzierte Mutante ist.

3. Bakterium nach Anspruch 1 oder 2, wobei das Bakterium im Vergleich zum Wildtyp Überexpression eines oder mehrer Gene, die Enzyme des Folatbiosynthesewegs codieren, zeigt.

4. Bakterium nach einem der vorherigen Ansprüche, wobei die Expression eines oder mehrerer Gene, ausgewählt aus den Genen, die Dihydrofolatsynthase, Fructokinase, Pyruvatoxidase und 6-Phospho-β-Glucosidase codieren, im Vergleich zum Wildtyp mindestens etwa 2-fach hochreguliert ist.

5. Bakterium nach einem der Ansprüche 1, 2 oder 4, wobei die Expression des Gens, das die Dihydroxyacetonphosphotransferase codiert, im Vergleich zum Wildtyp mindestens etwa 2-fach herunterreguliert ist.

6. Bakterium nach einem der vorherigen Ansprüche, wobei die Menge an Gesamtfolat des mutierten Bakteriums mindestens 10% höher als die des Wildtypstamms ist.

7. Bakterium nach einem der vorherigen Ansprüche, wobei das Bakterium von der Art *Lactobacillus plantarum, L. casei, L. reuteri, L. fermentum, L. acidophilus, L. crispatus, L. gasseri, L. johnsonii, L. paracasei, L. murinus, L. jensenii, L. salivarius, L. minutis, L. brevis, L. gallinarum, L. amylovorus, Lactococcus lactis, Streptococcus thermophilus, Leuconostoc mesenteroides, Lc. lactis, Pediococcus damnosus, P. acidilactici, P. parvulus, Bifidobacterium bifidum, B. longum, B. infantis, B. breve, B. adolescente, B. animalis, B. gallinarum, B. magnum* und *B. thermophilum* ist.

8. *Lactobacillus plantarum-Stamm* CBS 117120.

9. Zusammensetzung, die ein Bakterium nach einem der vorherigen Ansprüche umfasst.

10. Zusammensetzung nach Anspruch 9, wobei die Zusammensetzung eine Nahrungszusammensetzung oder Nahrungsergänzungszusammensetzung ist.

11. Zusammensetzung nach Anspruch 10, wobei die Nahrungszusammensetzung eine fermentierte Nahrungszusammensetzung ist.

12. Verwendung von Methotrexat zur Auswahl von Bakterien, die erhöhte intrazelluläre und erhöhte Gesamt-Folatspiegel haben.

13. Verfahren zur Auswahl von Bakterien, die erhöhte intrazelluläre und/oder Gesamt-Folatspiegel haben, wobei das Verfahren die Schritte umfasst:
(a) Wachstum der Bakterien auf oder in einem Medium, das Methotrexat umfasst, wobei das Medium nicht mit Folat-abhängigen Metaboliten ergänzt ist, wobei die Folat-abhängigen Metabolite Metabolite oder Vorläufer der Folatbiosysnthese oder des Folatkatabolismus sind, die geeignet sind, Methotrexatsensitivität einer Zelle zu maskieren, wenn sie im umgebenden Medium vorliegen,
(b) Auswählen von Bakterien, die eine Wachstumsrate (µ) von mindestens 0,1µ pro Stunde haben,
(c) Bestimmung des Folatspiegels der im Schritt (b) ausgewählten Bakterien und Auswahl der Bakterien, die im Vergleich zum Wildtyp erhöhte Folatspiegel haben,
(d) gegebenenfalls Wiederholung der Schritte (a), (b) und/oder (c).

14. Verfahren nach Anspruch 13, wobei das Medium mindestens 1,25 mg/l Methotrexat umfasst und wobei die Bakterien für mindestens 40 Stunden auf dem Medium wachsen.

## Revendications

1. Bactérie mutante, **caractérisée en ce que** la bactérie comprend une quantité augmentée de folate intracellulaire et/ou extracellulaire par rapport au type sauvage, et ayant un taux de croissance d'au moins 0,1µ par heure lorsqu'elle est cultivée sur un milieu comprenant 1,25 mg/l de méthotrexate et en l'absence de métabolites dépendant du folate, lesdits métabolites dépendant du folate étant des métabolites ou des précurseurs de la biosynthèse ou du catabolisme des folates qui sont capables de masquer la sensibilité au méthotrexate d'une cellule lorsqu'ils sont présents dans le milieu environnant.

2. Bactérie selon la revendication 1, dans laquelle ladite bactérie est un mutant d'origine naturelle ou un mutant induit.

3. Bactérie selon la revendication 1 ou 2, dans laquelle ladite bactérie présente une surexpression par rapport au type sauvage d'un ou de plusieurs gènes codant pour les enzymes de la voie de la biosynthèse du folate.

4. Bactérie selon l'une quelconque des revendications précédentes, dans laquelle l'expression d'un ou de plusieurs gènes sélectionnés parmi les gènes codant pour la dihydrofolate synthase, la fructokinase, la pyruvate oxydase et la 6-phospho-β-glucosidase est régulée à la hausse à raison d'au moins environ le double par rapport au type sauvage.

5. Bactérie selon la revendication 1, 2 ou 4, dans laquelle l'expression du gène codant pour la dihydroxyacétone phosphotransférase est régulée à la baisse à raison d'au moins environ le double par rapport au type sauvage.

6. Bactérie selon l'une quelconque des revendications précédentes, dans laquelle la quantité du folate total de la bactérie mutante est au moins de 10 % supérieure à celle de la souche de type sauvage.

7. Bactérie selon l'une quelconque des revendications précédentes, dans laquelle la bactérie est de l'espèce *Lactobacillus plantarum, L. casei, L. reuteri, L. fermentum, L. acidophilus, L. crispatus, L. gasseri, L. johnsonii, L. paracasei, L. murinus, L. jensenii, L. salivarius, L. minutis, L. brevis, L. gallinarum, L. amylovorus, Lactococcus lactis, Streptococcus thermophilus, Leuconostoc mesenteroides, Lc. Lactis, Pediococcus damnosus, P. acidilactici, P. parvulus, Bifidobacterium bifidum, B. longum, B. infantis, B. breve, B. adolescente, B. animalis, B. gallinarum, B. magnum* et *B. thermophilum.*

8. *Lactobacillus plantarum -* souche CBS 117120.

9. Composition comprenant une bactérie selon l'une quelconque des revendications précédentes.

10. Composition selon la revendication 9, dans laquelle la composition est une composition alimentaire ou une composition de complément alimentaire.

11. Composition selon la revendication 10, dans laquelle la composition alimentaire est une composition alimentaire fermentée.

12. Utilisation de méthotrexate pour la sélection de bactéries présentant des taux de folate intracellulaire et de folate total augmentés.

13. Procédé de sélection de bactéries présentant des taux de folate intracellulaire et/ou de folate total augmentés, ledit procédé comprenant les étapes consistant à :
(a) cultiver les bactéries sur ou dans un milieu comprenant du méthotrexate, où le milieu n'est pas supplémenté en métabolites dépendant du folate, lesdits métabolites dépendant du folate étant des métabolites ou des précurseurs de la biosynthèse ou du catabolisme des folates qui sont capables de masquer la sensibilité au méthotrexate d'une cellule lorsqu'ils sont présents dans le milieu environnant,
(b) sélectionner les bactéries ayant un taux de croissance (µ) au moins égal à 0,1 par heure,
(c) déterminer le taux de folate des bactéries sélectionnées à l'étape (b) et sélectionner les bactéries ayant des taux de folate augmentés par rapport au type sauvage,
(d) en option, répéter les étapes (a), (b) et/ou (c).

14. Procédé selon la revendication 13, dans lequel ledit milieu comprend au moins 1,25 mg/l de méthotrexate et dans lequel les bactéries sont cultivées sur ledit milieu pendant au moins 40 heures.
